# EUROPEAN PATENT SPECIFICATION

(11) **EP 3 860 688 B1**
(45) Date of publication and mention of the grant of the patent: **07.09.2022**
(21) Application number: 19753198.1
(22) Date of filing: 24.07.2019
(51) Int. Cl.: A61M 5/28, A61M 5/20, A61M 5/24, A61M 5/31, A61M 5/315

(54) **SYRINGE FOR RECONSTITUTING AND INJECTING A PHARMACEUTICAL SOLUTION**
SPRITZE ZUM REKONSTITUIEREN UND INJIZIEREN EINER PHARMAZEUTISCHEN LÖSUNG
SERINGUE DE RECONSTITUTION ET D'INJECTION D'UNE SOLUTION PHARMACEUTIQUE

(30) Priority: 05.10.2018 IT 201800009185
(43) Date of publication of application: 11.08.2021
(73) Proprietor: Orofino Pharmaceuticals Group SRL, 00193 Roma (IT)
(72) Inventor: OROFINO, Ernesto, 00193 Roma (IT)
(74) Representative: Perronace, Andrea
(86) International application number: PCT/IB2019/056316
(87) International publication number: WO 2020/070564

(56) References cited:
- EP-A1- 0 815 885
- EP-A2- 0 568 321
- WO-A1-88/02265
- WO-A1-2017/216651
- US-A- 3 807 119

## Description

The present invention relates to the technical field of syringes for injecting pharmaceutical products, in particular to prefilled multiple chamber syringes.

### Background art

Currently, sterile raw materials in powder form are mainly packed by active ingredient manufacturers either in aluminum containers or in plastic bags, generally made of polyethylene.

In order to be able to market them, manufacturers have demonstrated that each raw material, in addition to maintaining sterility, remains stable in the containers or bags used for a given period of time; in other words, such powders do not degrade for a predefined number of years when stored in such containers or bags.

The sterile raw materials are sold to the manufacturers of the finished pharmaceutical product, who fractionate them using known technologies, marketing the finished product in sterile powder form contained in a bottle. The bottle is accompanied by a glass vial containing the solvent.

The use of high-quality materials for the vial (glass) and of compatible materials for the sterile powder and the use of disposable syringes make this system very costly as a whole.

The use of syringes provided with a compartment for holding a cartridge, made of flexible material, containing pharmaceutical substances kept separate until the moment of use by a special soft septum, is known in the sector. An example of such devices is described in patent US2001/0047162. However, such known devices have some drawbacks. Indeed, due to accumulations and stagnations of the mixture or of the pharmaceutical substance in the corners of the cartridge, not the whole dose is delivered. This makes the dosage of the substance actually injected inaccurate and unreliable.

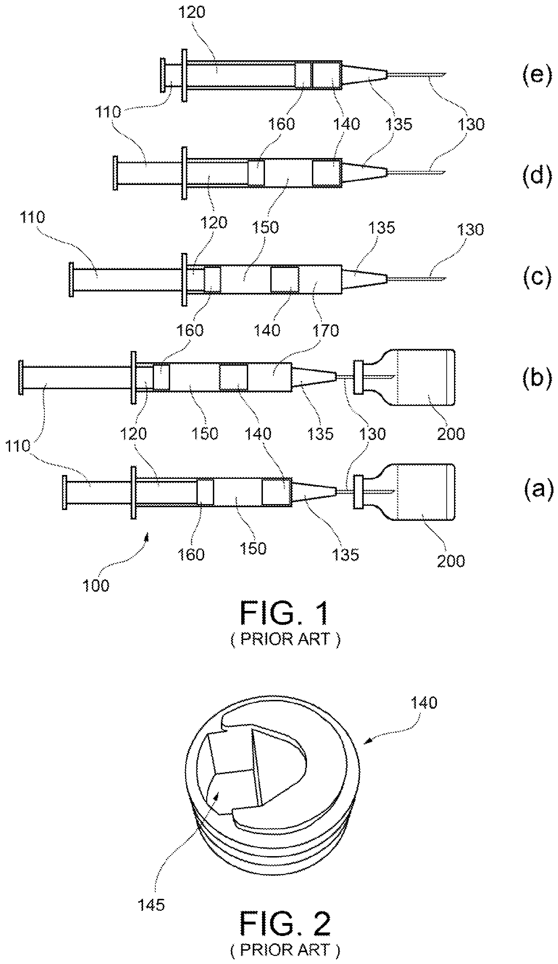

Other solutions, such as the Debiopass^{™} by Debiotech S.A. shown in Fig. 1, use a multi-chamber syringe 100, in which the chambers are defined by rubber plugs 140, 160 sliding in a transparent body 120. The initial configuration of the syringe 100 is shown in view (a), in which a chamber between the two plugs 140,160 is already filled with a first liquid 150. The plug 140 is at limit stop position in the body 120. The spout 135 of the syringe supports a needle 130, which is inserted into a vial 200 to aspirate a second liquid 170, visible in configuration (b). While it is being drawn, the second liquid creates the second chamber, pushing back the plug 140 and therefore also the plug 160. The plunger 110 is thus in the maximum protrusion position from the body 120. By removing the needle 130 from the vial 200, the syringe can be used in free state in (c) to administer the two liquids to a patient. The administration of the liquid 170 adjacent to the spout 135 leads to the state in view (d), in which the plug 140 is now in end stop position. By pushing the piston 110 further, the first liquid 150 is injected until the plug 160 comes into contact with the plug 140. In this manner, two liquids were successively injected into the patient with a single injection.

Figure 2 shows a known plug to be used in the multi-chamber (dynamic) syringe in Fig. 1, in particular as plug 140. A (non-through) cavity 145 which is initially filled with air can be noted. The liquid, while pushing the plunger 110, passes outside the plug and presses it in the direction indicated by the arrow in the figure, because of the presence of the cavity 145 which offers less resistance than the full zone. This creates the side passage for the fluid which is consequently dispensed. Such a plug allows the liquid not to be in initial contact with the spout 135.

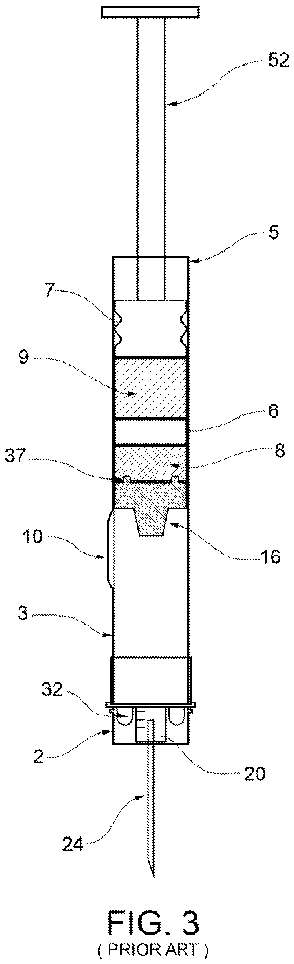

A further solution is shown in Fig. 3, taken from document WO2017216651 A1 by the Applicant, which, unlike the solution in Fig. 1, does not draw a liquid, but instead reconstitutes two liquids or a liquid and a powder previously introduced in respective chambers defined by movable plugs 6,7,16, to obtain an injectable solution in a single chamber. For such a purpose, there is a side bypass duct 10, which in the initial state of use of the syringe is located beyond the movable plugs. Furthermore, at the front end 2 there is a liquid storage cavity 20,32 of limited capacity. The liquid in excess is expelled before being able to use the syringe by injecting the solution of the storage cavity. For this reason, the plugs used in this syringe must all be movable and different from the plug in Fig. 3. Furthermore, the initial position of the plugs beyond the bypass allows prefilling the syringe with a very limited amount of the two substances. The stroke of the syringe plunger being equal, a much smaller amount of medicinal fluid will be administered than with other single chamber syringes.

One of the prior art which emerged in application WO2017216651 A1, i.e. European patent application EP0568321 A2, shows solutions similar to the above, in which the front plug must be pierced by a double tip syringe needle or a second bypass, which bypasses the front plug itself, is used. In the first case, the piercing operation is not sufficiently safe; in the second case, the construction of a second bypass is complicated. In both cases, structures and elements must be introduced which require a more articulated production line and therefore the product will be more expensive as a whole, while its operation will be far from optimal.

### Scope and object of the invention

It is the object of the present invention to solve the problems of the prior art by taking the needs of the sector into account.

In particular, it is the object of the present invention to provide a device for delivering pharmaceutical substances which allows an optimal operation by means of a simple and inexpensive system.

The present invention relates to a prefilled injection device according to one or more of the appended claims.

### Detailed description of examples of preferred embodiments of the invention

### List of drawings

The invention will now be described by way of example, with particular reference to the drawings of the accompanying figures, in which:
- figure 1 shows the different and progressive states of use of a double chamber syringe for the subsequent administration of two liquids, according to the prior art;
- figure 2 shows a sliding plug used in the syringe of Fig. 1, according to the prior art;
- figure 3 shows a double chamber syringe for the pre-reconstruction of a solution and subsequent administration of the solution, according to the known art;
- figure 4 shows the different and progressive states of use of a double chamber syringe for the pre-reconstruction of a solution and subsequent administration of the solution, according to the present invention;
- figure 5 shows the same plug as Fig. 2, this time used in the device of Fig. 4; and
- figure 6 shows the same embodiment as figure 4, but with the liquids/powder in motion.

It is worth noting that hereinafter elements of different embodiments may be combined together to provide further embodiments without limitations respecting the technical concept of the invention, as a person skilled in the art will effortlessly understand from the description.

The present description also makes reference to the prior art for its implementation, with regard to the detail features which are not described, such as, for example, elements of minor importance usually used in the prior art in solutions of the same type.

When an element is introduced, it is always understood that there may be "at least one" or "one or more".

When a list of elements or features are listed in this description, it is understood that the finding according to the invention "comprises" or alternatively "consists of" such elements.

### Embodiments

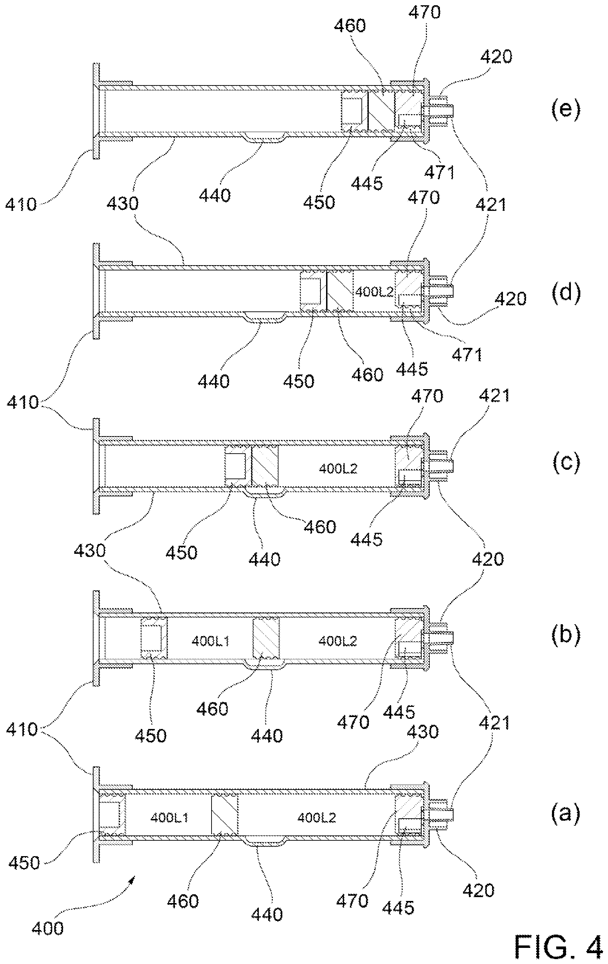

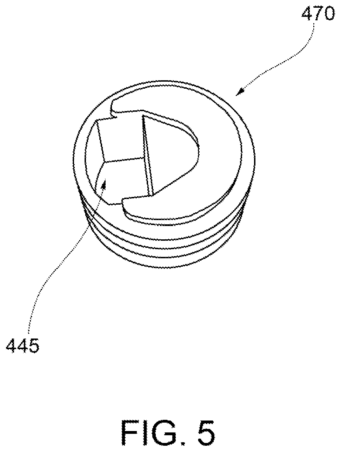

With reference to figs. 4 and 5, the prefilled injection device 400 comprises, according to an embodiment of the present description, a typical tubular containment body 430, which extends along an axis (not shown) between a front-end element 420 and a rear end element 410. It is apparent that, as in all syringes, the front-end element 420 is a partial closure element, with a spout 421 to which a needle or a small tube (not shown) is connected for administering or transferring the liquid or solution into the syringe.

Since this is a device in which the substances are already in the syringe and must be mixed, there are a first plug 470 and a second plug 460 arranged inside the tubular body 430 of the syringe, so as to delimit therebetween a second containment chamber 400L2 in the tubular body 430 itself containing a second liquid substance. At the same time, there is a third plug 450 connected to a syringe piston (not shown) and forming a first containment chamber 400L1 with said second plug 460 in the tubular body 430. The second containment chamber 400L2 contains a second substance, either solid or liquid, e.g. in powder form.

The second plug 460 hermetically separates the first containment chamber 400L1 from the second containment chamber 400L2. The second plug 460 and the third plug 450 are configured and adapted to slide inside the tubular body 430.

It is further apparent that, as in the traditional syringes of this type, there is at least one bypass channel 440, initially in a closed state and adapted to be taken into an opened state for mixing said first and said second substance. The bypass channel may be formed in various manners in the syringe.

According to the invention, and contrarily to the mentioned known art, in the prefilled injection device or syringe 400 the first plug 470 is arranged in a fixed position in contact with or near said front end element 420 along the axial direction. "Proximity" means a distance (either minimum or not) of construction convenience which can be easily determined according to the case.

It is worth noting here that the solution of application WO2017216651 A1 cannot use such a plug in fixed position, because such a device must store a predetermined amount of reconstituted liquid and must be able to move the plugs to allow the administration thereof. Furthermore, the bypass cannot be placed, in the initial state, between the two plugs, but both plugs must stay above the bypass, thereby limiting the amount of substances in the syringe.

By putting a fixed plug into abutment at the front end of the syringe as shown, a passage for the liquid is needed, and it is desirable that this does not complicate the constitution of the syringe itself.

This is achieved, according to an aspect of the present description, with a plug deformable under the pressure of the liquid which is pushed by the action of the plunger or piston of the syringe 400. In general, one-way passage means may be used, e.g. such as one-way valves. More in general, liquid communication means with said front end element 420 will be provided with/in said first plug.

According to a preferred embodiment, the first plug 470 is provided with a non-through cavity 445 which is offset with respect to said axis and facing towards said front end element 420 (the cavity 445 of 470 is facing towards 420, because if it were facing 410 it would be filled with solution, thereby making the plug stick on the walls of 430, because it is filled with liquid). This allows the plug to contract at least partially perpendicularly to said axis, and thus to create a channel 471 with the body 430 up to the spout 421 of the end element 420.

Fig. 5 shows an embodiment of the plug 470 with cavity 445, according to the invention.

The deformability, that is the narrowing of the plug 470 can be achieved by means of various inner configurations of the plug itself, e.g. by providing inner elements which can collapse or break (and in this case the parts which will break will remain advantageously coupled to the rest of the structure of the plug) under liquid pressure.

According to an embodiment of the present invention, the tubular body 430 comprises an inner wall provided with a recess adapted to define the bypass channel 440. In the initial configuration of the plugs, the bypass channel is positioned between the first plug 470 and the second plug 460.

According to a preferred aspect of the present description, the length of the bypass channel 440 is larger than the total length of the second plug 460, but smaller than the sum of the lengths of the plugs 450 and 460 (to prevent the liquid from flowing back once the third one 450 reaches the second one 460).

Alternatively, the bypass channel may be provided in the second plug 460, e.g. with a shrinkage following a collapse of the inner structure, or with an appropriate one-way valve (e.g. breakable membrane).

From the point of view of the materials of the syringe 400, according to the present description, the front-end element 420 can be made of plastic material and/or made in one piece with the tubular body 430. Similarly, the rear end element 410 may be made of plastic material and/or made in one piece with the tubular body. The tubular body may be made of plastic or glass, for example.

From the point of view of the substances present from the beginning in the syringe, the first liquid substance is a solvent for injectable use and the second substance may be an active substance or a highly active substance, e.g. an antibiotic, or a beta-lactam antibiotic (Cephalosporin and/or Penicillin antibiotic), or a cytotoxic anticancer substance, or a hormone, or a biological preparation, or a biotechnological product, or a monoclonal antibody, or a protein, or a vaccine, or an anesthetic.

Turning to the steps of operation of the device according to the present description, Fig. 4(a) shows the starting state of the device as described above, in which the plunger is not shown for ease of representation. The plunger can be connected to the third plug 450.

In Fig. 4(b), the plunger starts moving the plug 450 and, by means of the liquid of the chamber 400L1, also the plug 460 towards the zone of the bypass 440. Again in Fig. 4(b), the bypass is activated because of the position of the plug 460 inside the bypass itself, thereby allowing the liquid to pass from 400L1 to 400L2.

In Fig. 4(c), once the bypass is activated because of the position of 460 inside the bypass itself, 450 and 460 end up touching each other, the liquid being passed either entirely or almost totally from chamber 400L1 to chamber 400L2, thereby reconstituting itself with the second substance.

At this point, again with further push of the piston and thus of the plug 450, the second chamber 400L2 is reduced to Fig. 4(d) and substantially or totally canceled in the final state of Fig. 4(e). This is possible by virtue of the fluid communication means comprised in or constituting the front plug 470, which allow the reconstituted substance to reach the spout 421 and from there a needle or a tube or other fluid connection to a third body (e.g. the body of a patient who is injected with the syringe of the present description).

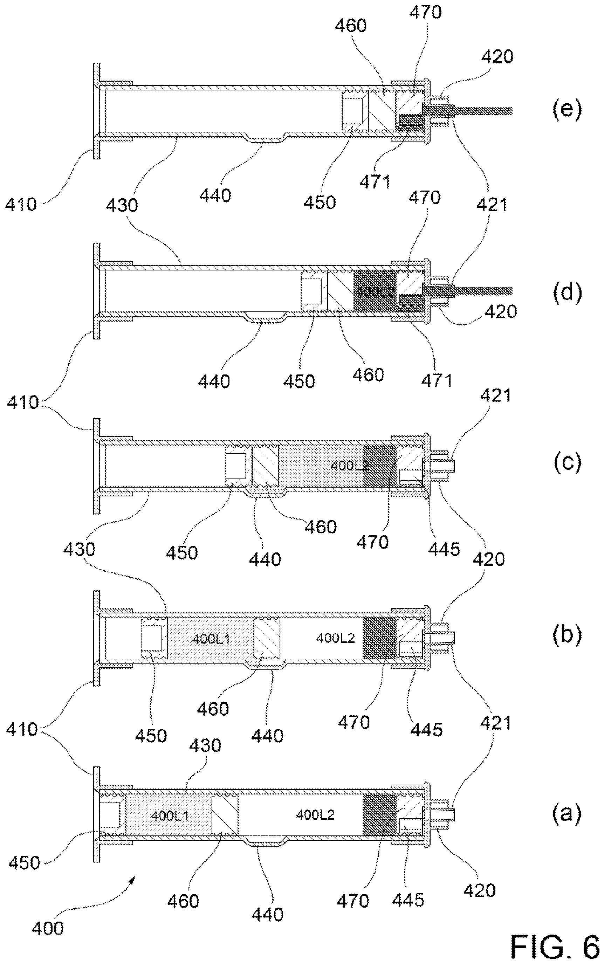

Fig. 6 shows the injection sequence as in Fig. 4 but with the various materials indicated in the syringe 400 with different patterns.

### Advantages of the invention

As explained above, among the advantages obtained with the device of the present description, there is the simple structure of the syringe with maximization of the substances to be injected at the same time. The substances to be (reconstituted and) injected being equal, smaller syringes than those of the prior art can therefore be made.

For this reason, but also because of the general shape similar to that of the prior art, and for the simplicity of the characterizing elements, production costs are minimized and operation is optimal.

Hereto, we have described the preferred embodiments and suggested some variants of the present invention, but it is understood that a person skilled in the art can make modifications and changes without departing from the respective scope of protection, as defined by the appended claims.

## Claims

1. A prefilled injection device (400), comprising:
- a tubular containment body (430), which extends along an axis between a front end element (420) and a rear end element (410), the front element (420) comprising a spout (421) to which a needle is connectable;
- a first (470) and a second plug (460) arranged inside the tubular body (430), so as to delimit therebetween a second containment chamber (400L2) in the tubular body (430) containing a second either solid or liquid substance;
- a third plug (450) connected to a syringe piston and forming a first containment chamber (400L1) with said second plug (460) in the tubular body (430), the first containment chamber (400L1) containing a first liquid substance;
- at least one bypass channel (440), configured to assume alternatively a closed state or an opened state;
wherein:
- the first plug (470) is arranged in a fixed position either near or in contact with said front end element (420) along said axis;
- the second plug (460) hermetically separates the first containment chamber (400L1) from the second containment chamber (400L2);
- the second (460) and third (450) plugs are configured and adapted to slide inside the tubular body (430);
- the tubular body (430) comprises an inner wall provided with a recess defining said at least one bypass channel (440), wherein the at least one bypass channel is positioned between the first plug (470) and the second plug (460) in an initial configuration of the first and second plug; the prefilled injection device (400) **being characterized in that** said first plug (470) comprises liquid communication means for passing, upon a movement of the syringe piston towards the front element (420), from a first closed state to a second opened state.

2. A prefilled injection device (400), according to claim 1, wherein the length of the at least one bypass channel (440) is larger than the total length of the second plug (460) but smaller than the sum of the lengths of the third (450) and second plug (460).

3. A prefilled injection device (400), comprising:
- a tubular containment body (430), which extends along an axis between a front end element (420) and a rear end element (410), the front element (420) comprising a spout (421) to which a needle is connectable;
- a first (470) and a second plug (460) arranged inside the tubular body (430), so as to delimit therebetween a second containment chamber (400L2) in the tubular body (430) containing a second either solid or liquid substance;
- a third plug (450) connected to a syringe piston and forming a first containment chamber (400L1) with said second plug (460) in the tubular body (430), the first containment chamber (400L1) containing a first liquid substance;
- at least one bypass channel (440), configured to assume alternatively a closed state or an opened state;
Wherein:
- the first plug (470) is arranged in a fixed position either near or in contact with said front end element (420) along said axis;
- the second plug (460) hermetically separates the first containment chamber (400L1) from the second containment chamber (400L2);
- the second (460) and third (450) plugs are configured and adapted to slide inside the tubular body (430); the prefilled injection device (400) being **characterized in that:**
- the at least one bypass channel is provided in the second plug (460);
- said first plug (470) comprises liquid communication means for passing, upon a movement of the syringe piston towards the front element (420), from a first closed state to a second opened state.

4. A prefilled injection device (400) according to claim 3, wherein the at least one bypass channel (440) consists of a one-way valve integrated in said second plug (460).

5. A prefilled injection device (400), according to claim 4, wherein the at least one bypass channel (440) consists of inner elements of the second plug (460) which are collapsible or breakable under liquid pressure.

6. A prefilled injection device (400), according to any claim 1-5, wherein said liquid communication means are constituted in the structure of said first plug (470), wherein the liquid communication means in the first plug (470) comprise a watertight closure with respect to said front end element (420) in said first closed state and said first plug (470) is shrunk perpendicularly to said axis in said second opened state, thereby forming a liquid communication channel (471) between said second containment chamber (400L2) and said front end element (420).

7. A prefilled injection device (400), according to claim 6, wherein said liquid communication means in the first plug (470) comprises a non-through cavity (445) which is offset with respect to said axis and facing towards said front end element (420).

8. A prefilled injection device (400) according to one or more of the preceding claims, wherein the front end element (420) is made of plastic material and/or made in one piece with the tubular body (430).

9. A prefilled injection device (400) according to one or more of the preceding claims, wherein the rear end element (410) is made of plastic material and/or made in one piece with the tubular body (430).

10. A prefilled injection device (400) according to one or more of the preceding claims, wherein the tubular body (430) is made of plastic or glass material.

11. A prefilled injection device (400) according to one or more of the preceding claims, wherein the first liquid substance is a solvent for injectable use and the second substance is an active substance or a highly active substance, e.g. an antibiotic, or a beta-lactam antibiotic (Cephalosporin and/or Penicillin antibiotic), or a cytotoxic anticancer substance, or a hormone, or a biological preparation, or a biotechnological product, or a monoclonal antibody, or a protein, or a vaccine, or an anesthetic.

## Patentansprüche

1. Vorgefüllte Injektionsvorrichtung (400), umfassend:
- einen röhrenförmigen Behälterkörper (430), der sich entlang einer Achse zwischen einem vorderen Endelement (420) und einem hinteren Endelement (410) erstreckt, wobei das vordere Element (420) eine Tülle (421) umfasst, an die eine Nadel anschließbar ist;
- einen ersten (470) und einen zweiten Stopfen (460), die innerhalb des röhrenförmigen Körpers (430) angeordnet sind, um dazwischen eine zweite Einschlusskammer (400L2) im röhrenförmigen Körper (430) zu begrenzen, die eine zweite entweder feste oder flüssige Substanz enthält;
- einen dritten Stopfen (450), der mit einem Spritzenkolben verbunden ist und mit dem zweiten Stopfen (460) im rohrförmigen Körper (430) eine erste Einschlusskammer (400L1) bildet, wobei die erste Einschlusskammer (400L1) eine erste flüssige Substanz enthält;
- mindestens einen Bypass-Kanal (440), der so konfiguriert ist, dass er alternativ einen geschlossenen oder einen geöffneten Zustand annimmt;
wobei:
- der erste Stopfen (470) in einer festen Position entweder in der Nähe oder in Kontakt mit dem vorderen Endelement (420) entlang der Achse angeordnet ist;
- der zweite Stopfen (460) die erste Einschlusskammer (400L1) hermetisch von der zweiten Einschlusskammer (400L2) trennt;
- der zweite (460) und der dritte (450) Stopfen konfiguriert und angepasst sind, um innerhalb des röhrenförmigen Körpers (430) zu gleiten;
- der rohrförmige Körper (430) eine Innenwand umfasst, die mit einem Ausschnitt versehen ist, der den mindestens einen Bypass-Kanal (440) definiert, wobei der mindestens eine Bypass-Kanal in einer anfänglichen Konfiguration des ersten und zweiten Stopfens zwischen dem ersten Stopfen (470) und dem zweiten Stopfen (460) positioniert ist;
die vorgefüllte Injektionsvorrichtung (400) **dadurch gekennzeichnet ist, dass** der erste Stopfen (470) Flüssigkeitsverbindungsmittel umfasst, um bei einer Bewegung des Spritzenkolbens in Richtung des vorderen Elements (420) von einem ersten geschlossenen Zustand in einen zweiten geöffneten Zustand überzugehen.

2. Vorgefüllte Injektionsvorrichtung (400) nach Anspruch 1, wobei die Länge des mindestens einen Bypass-Kanals (440) größer ist als die Gesamtlänge des zweiten Stopfens (460), aber kleiner als die Summe der Längen des dritten (450) und zweiten Stopfens (460).

3. Vorgefüllte Injektionsvorrichtung (400), umfassend:
- einen röhrenförmigen Behälterkörper (430), der sich entlang einer Achse zwischen einem vorderen Endelement (420) und einem hinteren Endelement (410) erstreckt, wobei das vordere Element (420) eine Tülle (421) umfasst, an die eine Nadel anschließbar ist;
- einen ersten (470) und einen zweiten Stopfen (460), die innerhalb des röhrenförmigen Körpers (430) angeordnet sind, um dazwischen eine zweite Einschlusskammer (400L2) im röhrenförmigen Körper (430) zu begrenzen, die eine zweite entweder feste oder flüssige Substanz enthält;
- einen dritten Stopfen (450), der mit einem Spritzenkolben verbunden ist und mit dem zweiten Stopfen (460) im rohrförmigen Körper (430) eine erste Einschlusskammer (400L1) bildet, wobei die erste Einschlusskammer (400L1) eine erste flüssige Substanz enthält;
- mindestens einen Bypass-Kanal (440), der so konfiguriert ist, dass er alternativ einen geschlossenen oder einen geöffneten Zustand annimmt;
wobei:
- der erste Stopfen (470) in einer festen Position entweder in der Nähe oder in Kontakt mit dem vorderen Endelement (420) entlang der Achse angeordnet ist;
- der zweite Stopfen (460) die erste Einschlusskammer (400L1) hermetisch von der zweiten Einschlusskammer (400L2) trennt;
- der zweite (460) und der dritte (450) Stopfen konfiguriert und angepasst sind, um innerhalb des röhrenförmigen Körpers (430) zu gleiten;
wobei die vorgefüllte Injektionsvorrichtung (400) **dadurch gekennzeichnet ist, dass**:
- der mindestens eine Bypass-Kanal im zweiten Stopfen (460) bereitgestellt ist;
- wobei der erste Stopfen (470) Flüssigkeitsverbindungsmittel umfasst, um bei einer Bewegung des Spritzenkolbens in Richtung des vorderen Elements (420) von einem ersten geschlossenen Zustand in einen zweiten geöffneten Zustand überzugehen.

4. Vorgefüllte Injektionsvorrichtung (400) nach Anspruch 3, wobei der mindestens eine Bypass-Kanal (440) aus einem in den zweiten Stopfen (460) integrierten Einwegventil besteht.

5. Vorgefüllte Injektionsvorrichtung (400) nach Anspruch 4, wobei der mindestens eine Bypass-Kanal (440) aus inneren Elementen des zweiten Stopfens (460) besteht, die unter Flüssigkeitsdruck kollabierbar oder zerbrechlich sind.

6. Vorgefüllte Injektionsvorrichtung (400) nach einem der Ansprüche 1-5, wobei die Flüssigkeitsverbindungsmittel in der Struktur des ersten Stopfens (470) gebildet sind, wobei die Flüssigkeitsverbindungsmittel im ersten Stopfen (470) einen wasserdichten Verschluss in Bezug auf das vordere Endelement (420) in dem ersten geschlossenen Zustand umfassen und der erste Stopfen (470) senkrecht zu der Achse in dem zweiten geöffneten Zustand geschrumpft ist, dadurch einen Flüssigkeitsverbindungskanal (471) zwischen der zweiten Einschlusskammer (400L2) und dem vorderen Endelement (420) bildend.

7. Vorgefüllte Injektionsvorrichtung (400) nach Anspruch 6, wobei das Flüssigkeitsverbindungsmittel im ersten Stopfen (470) einen nicht durchgehenden Hohlraum (445) umfasst, der in Bezug auf die Achse versetzt ist und dem vorderen Endelement (420) zugewandt ist.

8. Vorgefüllte Injektionsvorrichtung (400) nach einem oder mehreren der vorhergehenden Ansprüche, wobei das vordere Endelement (420) aus Kunststoffmaterial besteht und/oder in einem Stück mit dem rohrförmigen Körper (430) hergestellt ist.

9. Vorgefüllte Injektionsvorrichtung (400) nach einem oder mehreren der vorhergehenden Ansprüche, wobei das hintere Endelement (410) aus Kunststoffmaterial besteht und/oder in einem Stück mit dem rohrförmigen Körper (430) hergestellt ist.

10. Vorgefüllte Injektionsvorrichtung (400) nach einem oder mehreren der vorangehenden Ansprüche, wobei der röhrenförmige Körper (430) aus Kunststoff oder Glasmaterial hergestellt ist.

11. Vorgefüllte Injektionsvorrichtung (400) nach einem oder mehreren der vorhergehenden Ansprüche, wobei die erste flüssige Substanz ein Lösungsmittel zur injizierbaren Verwendung ist und die zweite Substanz eine aktive Substanz oder eine hochaktive Substanz ist, z. B. ein Antibiotikum oder ein Beta-Lactam-Antibiotikum (Cephalosporin- und/oder Penicillin-Antibiotikum) oder eine zytotoxische Anti-Krebs-Substanz oder ein Hormon oder eine biologische Zubereitung oder ein biotechnologisches Produkt oder ein monoklonaler Antikörper oder ein Protein oder ein Impfstoff oder ein Anästhetikum.

## Revendications

1. Dispositif d'injection prérempli (400) comprenant :
- un corps tubulaire de confinement (430) qui s'étend le long d'un axe entre un élément d'extrémité avant (420) et un élément d'extrémité arrière (410), l'élément avant (420) comprenant un bec (421) auquel peut être reliée une aiguille ;
- un premier (470) et un deuxième bouchon (460), agencés à l'intérieur du corps tubulaire (430) afin de délimiter entre eux une seconde chambre de confinement (400L2) dans le corps tubulaire (430) contenant une seconde substance solide ou liquide ;
- un troisième bouchon (450) relié à un piston de seringue et formant une première chambre de confinement (400L1) avec ledit deuxième bouchon (460) dans le corps tubulaire (430), la première chambre de confinement (400L1) contenant une première substance liquide ;
- au moins un canal de dérivation (440), conçu pour adopter alternativement un état fermé ou un état ouvert ;
dans lequel :
- le premier bouchon (470) est agencé en position fixe soit près dudit élément d'extrémité avant (420) soit en contact avec ce dernier le long dudit axe ;
- le deuxième bouchon (460) sépare hermétiquement la première chambre de confinement (400L1) de la seconde chambre de confinement (400L2) ;
- les deuxième (460) et troisième (450) bouchons sont conçus et adaptés pour coulisser à l'intérieur du corps tubulaire (430) ;
- le corps tubulaire (430) comprend une paroi intérieure comportant un évidement définissant ledit au moins un canal de dérivation (440), dans lequel l'au moins un canal de dérivation est positionné entre le premier bouchon (470) et le deuxième bouchon (460) dans une configuration initiale des premier et deuxième bouchons ;
le dispositif d'injection prérempli (400) **se caractérisant en ce que** ledit premier bouchon (470) comprend des moyens de communication de liquide pour passer, lors d'un déplacement du piston de seringue vers l'élément avant (420), d'un premier état fermé à un second état ouvert.

2. Dispositif d'injection prérempli (400) selon la revendication 1, dans lequel la longueur de l'au moins un canal de dérivation (440) est supérieure à la longueur totale du deuxième bouchon (460) mais inférieure à la somme des longueurs du troisième (450) et du deuxième bouchon (460).

3. Dispositif d'injection prérempli (400) comprenant :
- un corps tubulaire de confinement (430) qui s'étend le long d'un axe entre un élément d'extrémité avant (420) et un élément d'extrémité arrière (410), l'élément avant (420) comprenant un bec (421) auquel peut être reliée une aiguille ;
- un premier (470) et un deuxième bouchon (460), agencés à l'intérieur du corps tubulaire (430) afin de délimiter entre eux une seconde chambre de confinement (400L2) dans le corps tubulaire (430) contenant une seconde substance solide ou liquide ;
- un troisième bouchon (450) relié à un piston de seringue et formant une première chambre de confinement (400L1) avec ledit deuxième bouchon (460) dans le corps tubulaire (430), la première chambre de confinement (400L1) contenant une première substance liquide ;
- au moins un canal de dérivation (440), conçu pour adopter alternativement un état fermé ou un état ouvert ;
dans lequel :
- le premier bouchon (470) est agencé en position fixe soit près dudit élément d'extrémité avant (420) soit en contact avec ce dernier le long dudit axe ;
- le deuxième bouchon (460) sépare hermétiquement la première chambre de confinement (400L1) de la seconde chambre de confinement (400L2) ;
- les deuxième (460) et troisième (450) bouchons sont conçus et adaptés pour coulisser à l'intérieur du corps tubulaire (430) ;
le dispositif d'injection prérempli (400) **se caractérisant en ce que** :
- l'au moins un canal de dérivation est situé dans le deuxième bouchon (460) ;
- ledit premier bouchon (470) comprend des moyens de communication de liquide pour passer, lors d'un mouvement du piston de seringue vers l'élément avant (420), d'un premier état fermé à un second état ouvert.

4. Dispositif d'injection prérempli (400) selon la revendication 3, dans lequel l'au moins un canal de dérivation (440) est constitué d'une vanne unidirectionnelle intégrée audit deuxième bouchon (460).

5. Dispositif d'injection prérempli (400) selon la revendication 4, dans lequel l'au moins un canal de dérivation (440) est constitué d'éléments internes du deuxième bouchon (460) qui sont pliables ou cassables sous la pression de liquide.

6. Dispositif d'injection prérempli (400) selon l'une quelconque des revendications 1 à 5, dans lequel lesdits moyens de communication de liquide font partie de la structure dudit premier bouchon (470), dans lequel les moyens de communication de liquide du premier bouchon (470) comprennent une fermeture étanche par rapport audit élément d'extrémité avant (420) dans ledit premier état fermé et ledit premier bouchon (470) est rétracté perpendiculairement sur ledit axe dans ledit second état ouvert, ce qui forme un canal de communication de liquide (471) entre ladite seconde chambre de confinement (400L2) et ledit élément d'extrémité avant (420).

7. Dispositif d'injection prérempli (400) selon la revendication 6, dans lequel lesdits moyens de communication de liquide du premier bouchon (470) comprennent une cavité non traversante (445) décalée par rapport audit axe et tournée vers ledit élément d'extrémité avant (420).

8. Dispositif d'injection prérempli (400) selon une ou plusieurs des revendications précédentes, dans lequel l'élément d'extrémité avant (420) est en matière plastique et/ou d'une seule pièce avec le corps tubulaire (430).

9. Dispositif d'injection prérempli (400) selon une ou plusieurs des revendications précédentes, dans lequel l'élément d'extrémité arrière (410) est en matière plastique et/ou d'une seule pièce avec le corps tubulaire (430).

10. Dispositif d'injection prérempli (400) selon une ou plusieurs des revendications précédentes, dans lequel le corps tubulaire (430) est en matière plastique ou en verre.

11. Dispositif d'injection prérempli (400) selon une ou plusieurs des revendications précédentes, dans lequel la première substance liquide est un solvant à usage injectable et la seconde substance est une substance active ou une substance hautement active, par exemple un antibiotique ou un antibiotique bêta-lactamine (céphalosporine et/ou pénicilline), une substance anticancéreuse cytotoxique, une hormone, une préparation biologique, un produit biotechnologique, un anticorps monoclonal, une protéine, un vaccin ou un anesthésique.
